# EUROPEAN PATENT SPECIFICATION

(11) **EP 4 012 029 B1**
(45) Date of publication and mention of the grant of the patent: **29.05.2024**
(21) Application number: 20853500.5
(22) Date of filing: 07.08.2020
(51) Int. Cl.: C12N 15/11, C12N 15/10, C40B 40/06, C40B 80/00, C12Q 1/6869, C12Q 1/6806

(54) **METHOD FOR CAPTURING NUCLEIC ACID MOLECULE, PREPARATION METHOD FOR NUCLEIC ACID LIBRARY, AND A SEQUENCING METHOD**
VERFAHREN ZUM EINFANGEN VON NUKLEINSÄUREMOLEKÜLEN, VERFAHREN ZUR HERSTELLUNG EINER NUKLEINSÄUREBIBLIOTHEK UND SEQUENZIERUNGSVERFAHREN
PROCÉDÉ DE CAPTURE D'UNE MOLÉCULE D'ACIDE NUCLÉIQUE, PROCÉDÉ DE PRÉPARATION D'UNE BANQUE D'ACIDES NUCLÉIQUES ET PROCÉDÉ DE SÉQUENÇAGE

(30) Priority: 09.08.2019 CN 201910733022; 09.08.2019 CN 201910733033
(43) Date of publication of application: 15.06.2022
(73) Proprietor: GeneMind Biosciences Company Limited, Shenzhen, Guangdong 518023 (CN)
(72) Inventor: LI, Gailing, Shenzhen City, Guangdong 518023 (CN); GAN, Guangli, Shenzhen City, Guangdong 518023 (CN); LI, Yan, Shenzhen City, Guangdong 518023 (CN); LUO, Mingjie, Shenzhen City, Guangdong 518023 (CN); ZHANG, Meng, Shenzhen City, Guangdong 518023 (CN); SUN, Lei, Shenzhen City, Guangdong 518023 (CN)
(74) Representative: Schiweck Weinzierl Koch Patentanwälte Partnerschaft mbB
(86) International application number: PCT/CN2020/107695
(87) International publication number: WO 2021/027706

(56) References cited:
- WO-A1-2009/072972
- WO-A1-2015/117040
- WO-A1-2017/182578
- WO-A1-2018/050722
- WO-A1-2018/053362
- WO-A1-2018/114706
- WO-A1-2019/126803
- CN-A- 103 774 242
- CN-A- 105 793 438
- CN-A- 106 048 009
- CN-A- 110 438 121
- CN-A- 110 628 880
- US-B2- 8 133 667

## Description

### CROSS REFERENCE TO RELATED APPLICATIONS

The present application claims priority and benefits to Chinese Patent Application Nos. 201910733022.1 and 201910733033.x filed with China National Intellectual Property Administration on Aug. 9, 2019.

### TECHNICAL FIELD

The present application relates to the field of molecular biology, and in particular to a method for capturing a nucleic acid molecule, a method for preparing a nucleic acid library and a sequencing method.

### BACKGROUND

Sequence capture is a technique in which a probe is designed for a specific region of a genome and used to selectively isolate or enrich a specific fragment of the genome. Common sequence capture methods include probe capture and PCR capture. Probe capture is generally used to capture a target sequence by means of the complementarity between the probe and the target sequence, and the target sequence captured is often a single-stranded sequence.

WO2015/117040 A1 discloses improved methods for processing DNA substrates.

WO2018/050722 A1 discloses methods for labelling nucleic acids.

WO2018/114706 A1 discloses single stranded circular DNA libraries for circular consensus sequencing.

WO2019/126803 A1discloses error removal using improved library preparation methods.

WO2017/182578 A1 discloses a method for generating a stranded RNA library.

WO2018/053362 A1 discloses methods of nucleic acid sample preparation.

In terms of library construction, next-generation sequencing and single-molecule sequencing often involve ligation of DNA fragments to a specific adapter to form a sequencing library. The type of adapters has a direct bearing on ligation efficiency, as well as the proportion of effective portion in the sequencing library. Different sequencing platforms require different sequencing adapters, and each platform requires optimization of the library construction method and the adapter used. For example, in Illumina^{™} library construction, Y adapter and Bubble adapter have different advantages. In the field of sequencing, sample processing before on-machine sequencing, including nucleic acid processing, library construction, etc., needs further optimization to improve the efficiency of library construction and simplify the operation process.

### SUMMARY

The invention is defined by the claims.

The present application provides a method for capturing a target nucleic acid molecule, by which a double-stranded molecule, as well as a single-stranded molecule, can be directly captured; in addition, the probability of non-specific hybridization can be reduced, and thus the efficiency of hybridization with a target fragment (first strand) can be improved. The present application provides a method for preparing a nucleic acid library; the nucleic acid library prepared by the method can be more efficiently captured by a probe, and high-proportion effective data can be further obtained when the library is sequenced.

In a first aspect, the present application discloses a method for capturing a nucleic acid molecule, wherein the nucleic acid molecule comprises a first nucleic acid strand and a second nucleic acid strand that are complementary, and has at least one single-stranded end located on the first nucleic acid strand. The method comprises capturing the nucleic acid molecule with a probe, including hybridizing the probe with at least a portion of the first nucleic acid strand to obtain a hybrid complex, the at least a portion of the first nucleic acid strand comprising the single-stranded end.

It could be understood that the first nucleic acid strand and the second nucleic acid strand of the nucleic acid molecule are two different strands or two different parts of one strand, and when the nucleic acid molecule is one strand, the first nucleic acid strand and the second nucleic acid strand are linked together at one end of the double-stranded region to form a nucleic acid molecule having a single-stranded region at one end.

The single-stranded end may be located at the 3'-end and/or the 5'-end of the first nucleic acid strand. The single-stranded region could be complementary pairing to the probe. Optionally, the single-stranded end is located at the 3'-end of the first nucleic acid strand. It can be known from the complementary base pairing rule that, when the probe is paired with the first nucleic acid strand and the single-stranded end is located at the 3'-end of the first nucleic acid strand, the probe can be used as a primer, and the first nucleic acid strand can be used as a template for amplification.

Also, it could be understood that the probe paired with the first nucleic acid strand comprises an oligonucleotide strand, and when the oligonucleotide strand is located at one end of the probe, the other end of the probe may be a nucleotide sequence or other sequences, such as an amino acid sequence, and/or with other modifications, such as amino modifications and fluorescent molecule.

The oligonucleotide strand in the probe is designed based on such factors as the annealing temperature, nucleotide composition, length and internal secondary structure of the oligonucleotide strand. In one embodiment of the present application, the oligonucleotide strand has a length of 20-80 nt.

Further, the nucleotide at the 3'-end of the oligonucleotide strand may comprise a hydroxyl group (-OH), so that a nucleotide may incorporate to the 3'-end of the oligonucleotide strand by a phosphodiester bond.

In one embodiment, the probe is immobilized on a solid base selected from at least one of glass, plastic and magnetic beads. Thus, in one aspect, facilitates the separation and purification of a nucleic acid sequence captured by the probe, and in another aspect, enables other detections or operations, such as amplification and sequencing, on the nucleic acid sequence captured by the probe.

In one embodiment, the nucleic acid molecule is obtained by ligating a first nucleic acid fragment and a second nucleic acid fragment, wherein the first nucleic acid fragment and the second nucleic acid fragment are both double-stranded DNAs; the first nucleic acid fragment is a known sequence and comprises a first strand and a second strand that are complementary; and the single-stranded end is located on the first strand.

It could be understood that when the nucleic acid molecule is obtained by ligating the first nucleic acid fragment and the second nucleic acid fragment and the two nucleic acid fragments are different, the first nucleic acid strand of the nucleic acid molecule actually comprises the first strand of the first nucleic acid fragment and the first strand of the second nucleic acid fragment, and the second nucleic acid strand of the nucleic acid molecule actually comprises the second strand of the first nucleic acid fragment and the second strand of the second nucleic acid fragment; however, when the first nucleic acid fragment and the second nucleic acid fragment are the same, the first nucleic acid strand of the nucleic acid molecule comprises the first strand of the first nucleic acid fragment and the second strand of the second nucleic acid fragment, and the second nucleic acid strand of the nucleic acid molecule comprises the second strand of the first nucleic acid fragment and the first strand of the second nucleic acid fragment.

The nucleic acid fragments may be linked by blunt-end ligation and/or sticky-end ligation. For the nucleic acid fragments with blunt ends at both ends, ligation can be performed after T/A is added to the ends, which can improve ligation efficiency and ensure ligation directionality.

It will be understood that the nucleic acid fragments can be linked by chemical bonds, including but not limited to, phosphodiester bonds, and when the nucleic acid fragments are linked by phosphodiester bonds, the 5'-end of the first strand has a phosphate group. A natural nucleotide comprises a phosphate group and a hydroxyl group, which are chemically linked with phosphodiester bonds, so that the natural structural characteristics of the nucleotide strand can be retained in the ligation product.

In one embodiment, the 3'-end of the second strand has no hydroxyl group, and thus cannot be ligated to the second nucleic acid fragment by a phosphodiester bond. With this scheme, there would be two ligation products, one is, the second strand is linked to the second nucleic acid fragment by a non-phosphodiester bond, and the other is, the ligation product does not comprise a second strand. For these two ligation products, if used as PCR templates, the respective second strands cannot be effectively amplified; and if used as sequencing templates, the sequencing of invalid data can be reduced and the amount of valid sequencing data can be increased..

The melting temperature difference between the two complementary strands in the first nucleic acid fragment is at least 10 °C and is not greater than 80 °C, i.e., 80 °C ≥ Tm1 - Tm2 ≥ 10 °C, and 90 °C ≥ Tm1 ≥ 50 °C, where Tm1 is a melting temperature of the first strand, and Tm2 is a melting temperature of the second strand. An appropriate melting temperature difference is set between the two complementary strands in the first nucleic acid fragment may ensure that the first strand preferentially hybridizes to the probe after denaturation of the first nucleic acid fragment and during annealing. The probe may be complemently to all or part of the sequence of the first strand, and in a preferred embodiment, the entire sequence of the first strand is complemently to the probe. It could be understood that the above conditions are to set a appropriate annealing temperature to allow the first strand to preferentially hybridize with the probe; moreover, the pairing sequence of the first strand and the probe may allow the existence of mismatches, such as 0, 1, 2 or 3 or preferably not more than 5%..

In one embodiment of the present application, the Tm1 is 71 °C, and the Tm2 is 45.6 °C. After the melting temperatures of both strands are determined, the annealing temperature T for hybridization with the probe is selected: Tm2 - 5 °C ≤ T ≤ Tm1 - 5 °C. In one embodiment of the present application, the selected annealing temperature is 55 °C.

In one implementation of the present application, the method for capturing a nucleic acid molecule disclosed herein specifically comprises: linking a first nucleic acid fragment to a second nucleic acid fragment to obtain a nucleic acid molecule, denaturing to melt the nucleic acid molecule into single strands, rapidly cooling, for example, placing a solution containing the melt nucleic acid molecules on ice, and hybrid capture, wherein the first nucleic acid fragment and the second nucleic acid fragment are both double-stranded DNAs; the first nucleic acid fragment is a known sequence and comprises a first strand and a second strand that are complementary; and the nucleic acid molecule has at least one single-stranded end located on the first strand, wherein the 5'-end of the first strand has a phosphate group, the 3'-end of the second strand has no hydroxyl group, and the entire sequence of the first strand are complementarily paired with a probe used for the capture. The method can greatly improve the capture efficiency and eliminate the effect of the second strand on the capture.

When the nucleic acid molecule is complementarily bound to the probe, the binding between the two needs to be visually determined, particularly from the aspects of the binding efficiency and the binding positions, so as to determine whether the method is successful. When the probe is immobilized, the binding efficiency can be determined from the number of immobilized nucleic acid molecules. For this reason, in one preferred embodiment, the first strand carries an optically detectable label, preferably a fluorescent molecule, such as Cy3 and Cy5, at its 3'-end, so that the hybridization efficiency can be detected easily and visually.

In order to capture single-stranded nucleic acid molecules, in one example, double-stranded nucleic acid molecules are melted into single-stranded nucleic acid molecules, for example, by denaturation, and then the probe is hybridized with the single-stranded nucleic acid molecule to form a hybrid complex. The denatured nucleic acid molecule is complementarily bound to the probe in a hybridization solution.

By the capture method, in one aspect, double-stranded nucleic acid molecules can be captured, and in another aspect, single-stranded nucleic acid molecules can be captured. After the double-stranded nucleic acid molecules are captured by the method, in one aspect, the target double-stranded molecule can be purified and isolated, and in another aspect, a target sequence, such as a probe sequence of a nucleic acid fragment, can be synthesized from the formed hybrid complex in the presence of a polymerase for strand displacement reaction. After the single-stranded nucleic acid molecules are captured, the formed hybrid complex can be used for sequencing, thereby determining specific sequence information of a nucleic acid fragment.

The above method for capturing a nucleic acid molecule skillfully utilizes the characteristic that a double-stranded nucleic acid molecule has a single-stranded region, and thus can be used to effectively capture a double-stranded nucleic acid sequence, as well as a single-stranded nucleotide. By using the Tm relationship between the single-stranded region and the double-stranded region, an appropriate hybrid capture temperature is designed, so that the probability of hybridization of the second strand with the first strand is reduced while non-specific hybridization is reduced, thereby improving the hybridization efficiency of the probe with the first strand.

In a second aspect, the present application provides a hybrid complex obtained by the capture method described above. There are two types of hybrid complexes. The first type is formed by hybridization of a probe with an undenatured nucleic acid molecule (i.e., a double-stranded nucleic acid molecule), wherein the probe is paired with a single-stranded end to form a hybrid complex, which comprises three sequences, i.e., two sequences from the nucleic acid molecule and a probe sequence. The second type is formed by hybridization of a probe with a single-stranded nucleic acid molecule resulting from denaturation, wherein the probe is paired and hybridized with the sequence comprising at least a single-stranded end sequence to form a hybrid complex, which comprises two sequences, i.e., a sequence from the single-stranded nucleic acid molecule and a probe sequence.

In a third aspect, the present application provides a kit comprising the nucleic acid molecule described above. The kit further comprises a probe and/or a hybrid complex. The nucleic acid molecule in the kit comprises a first nucleic acid strand and a second nucleic acid strand that are complementary and has at least one single-stranded end located on the first nucleic acid strand, and the sequence of the probe in the kit is complementary to that of the single-stranded end on the first nucleic acid strand. This kit can be used for sequence hybrid capture experiments, control experiments of probe sequence hybrid capture experiments and sequencing.

In a fourth aspect, the present application provides use of the kit described above in sequence hybrid capture and/or sequencing. The kit can be used in a control group for quality control of the sequence hybrid capture.

In a fifth aspect, the present application provides a sequencing method comprising sequencing a hybrid complex obtained by the above method. In sequencing, when the hybrid complex is a complex comprising three sequences, a strand displacement DNA polymerase, such as Phi29 DNA polymerase, is adopted for sequencing; when the hybrid complex is a complex comprising two sequences, a DNA polymerase, such as Taq DNA polymerase, is commonly used for sequencing.

In a sixth aspect, the present application provides a method for preparing a nucleic acid library, which comprises linking a first nucleic acid fragment to a second nucleic acid fragment to obtain the nucleic acid library, wherein the first nucleic acid fragment and the second nucleic acid fragment are both double-stranded DNAs; the first nucleic acid fragment is a known sequence and comprises a first strand and a second strand that are complementary; the first nucleic acid fragment has a single-stranded end located at the 3'-end of the first strand; and 80 °C ≥ (Tm1 - Tm2) ≥ 10 °C and 90 °C ≥ Tm1 ≥ 50 °C, where Tm1 is a melting temperature of the first strand, and Tm2 is a melting temperature of the second strand.

In one embodiment of the present application, the Tm1 is 71 °C, and the Tm2 is 45.6 °C.

It could be understood that the nucleic acid fragments can be linked by chemical bonds, including but not limited to, phosphodiester bonds, and when the nucleic acid fragments are linked by phosphodiester bonds, the 5'-end of the first strand has a phosphate group. A natural nucleotide comprises a phosphate group and a hydroxyl group, which are chemically linked with phosphodiester bonds, so that the natural structural characteristics of the nucleotide strand can be retained in the ligation product.

In one embodiment, the 3'-end of the second strand has no hydroxyl group, and a second strand having such a characteristic is used to construct a nucleic acid library. The ligation products in the nucleic acid library are in two forms. In one form, the second strand is linked to the second nucleic acid fragment by a non-phosphodiester bond, and in the other form, the ligation product does not comprise a second strand. When the two strands of the ligation product in these two forms are used as a PCR substrate, the second strand cannot be effectively amplified, so that ineffective sequencing data can be reduced and effective sequencing data can be increased if the ligation product is used as a sequencing substrate.

When the nucleic acid library prepared by the above method for preparing a nucleic acid library is used for sequencing, particularly for single-molecule sequencing, the hybridization efficiency with the probe can be improved, thereby increasing the amount of target fragments for effective sequencing. In an embodiment of the present application, the first nucleic acid fragment of the present application and a blunt-end adapter are each used for constructing a nucleic acid library for probe capture hybridization, and the capture hybridization efficiencies are compared. The size of the nucleic acid libraries is determined by detecting fluorescent signals after the nucleic acid library fragments are captured by the probe. As can be seen from the results, the efficiency of the probe for capturing the nucleic acid library obtained by using the method for constructing a library disclosed herein is more than 2 times that of the probe for capturing the library constructed using the blunt-end adapter.

The constructed library has multiple uses, such as chip sequence hybridization and sequencing. When the nucleic acid library is complementarily bound to the probe, the binding between the two needs to be visually determined, particularly from the aspects of the binding efficiency and the binding positions, so as to determine whether the operation is successful. When the probe is immobilized, the binding efficiency and the binding positions can be determined from the number of immobilized nucleic acid molecules. For this reason, in one preferred embodiment, the first strand carries an optically detectable label, preferably a fluorescent molecule, such as Cy3 and Cy5, at its 3'-end, so that the hybridization efficiency can be detected easily and visually.

In the method for preparing a nucleic acid library, the nucleic acid fragments may be linked by blunt-end ligation and/or sticky-end ligation. For the nucleic acid fragments with blunt ends at both ends, ligation can be performed after T/A is added to the ends, which can improve ligation efficiency and ensure ligation directionality.

When two nucleic acid fragments are linked, the molar weight of the nucleic acid fragment of known sequence, i.e., the first nucleic acid fragment, is greater than that of the second nucleic acid fragment, thereby increasing the probability of linking the second nucleic acid fragment to the first nucleic acid fragment. It can be known from the optimization results of the molar concentration ratio of the two nucleic acid fragments based on a plurality of experiments that, the first nucleic acid fragment and the second nucleic acid fragment can be effectively linked when being mixed at a molar ratio of 50:1 to 500:1; when the molar ratio is 200:1, more than 90% of the second nucleic acid fragments is linked to the first nucleic acid fragments, and the self-linking rate of the second nucleic acid fragments (linking between the second nucleic acid fragments) is greatly reduced. In one implementation of the present application, the preparation method disclosed herein comprises: linking a first nucleic acid fragment to a second nucleic acid fragment to obtain the nucleic acid library, wherein the first nucleic acid fragment and the second nucleic acid fragment are both double-stranded DNAs; the first nucleic acid fragment is a known sequence and comprises a first strand and a second strand that are complementary; the first nucleic acid fragment has a single-stranded end located at the 3'-end of the first strand; and 80 °C ≥ (Tm1 - Tm2) ≥ 10 °C and 90 °C ≥ Tm1 ≥ 50 °C, where Tm1 is a melting temperature of the first strand, and Tm2 is a melting temperature of the second strand. The 3'-end of the second strand has no hydroxyl group, and the 5'-end of the first strand comprises a phosphate group, and the first strand can be linked to the second nucleic acid fragment by a phosphodiester bond. In a nucleic acid library constructed by this method, the ligation product does not comprise a second strand, or the second strand is linked to a second nucleic acid fragment by a non-phosphodiester bond. When the two strands of the ligation product in these two forms are used as a PCR substrate, the second strand cannot be effectively amplified, while ineffective sequencing data can be reduced and effective sequencing data can be increased if the ligation product is used as a sequencing substrate.

In a seventh aspect, the present application provides a nucleic acid library, which is the ligation product obtained by the above method for preparing a nucleic acid library, wherein the ligation product is a product obtained by directly linking the first nucleic acid fragment to the second nucleic acid fragment or a product obtained by subjecting the product obtained by direct ligation to n cycles (1 ≤ n ≤ 6, and n is an integer) of PCR amplification.

In an eighth aspect, the present application provides a method for capturing a nucleic acid molecule, comprising:
1) denaturing the nucleic acid library into single strands; and 2) hybridizing the single-stranded product obtained in step 1) with a probe to form a hybrid complex.

The probe comprises an oligonucleotide strand. The oligonucleotide strand at one end of the probe is used for complementary hybridization with a sequence in a nucleic acid molecule, and it will be understood that the other end of the probe may be a nucleotide sequence or other sequences, such as an amino acid sequence, with other modifications, such as amino modifications and fluorescent modifications. In one embodiment of the present application, the sequences of the probes are the same, i.e., there is only one type of probe on the chip surface; the probe is complementarily paired with the first strand of a first nucleic acid strand, or only complementarily paired with a single-stranded end of the first nucleic acid strand, and the nucleotide at the 3'-end comprises a hydroxyl group (-OH). It can be known from the complementary base pairing rule that, when the probe is paired with the first nucleic acid strand and the single-stranded end is located at the 3'-end of the first nucleic acid strand, the probe can be used as a primer, and the first nucleic acid strand can be used as a template for PCR amplification. Optionally, the oligonucleotide strand is a DNA strand.

The nucleic acid library comprises a first nucleic acid fragment and a second nucleic acid fragment that are linked and are both double-stranded DNAs; the first nucleic acid fragment is a known sequence and comprises a first strand and a second strand that are complementary; and the first nucleic acid fragment has a single-stranded end located at the 3'-end of the first strand. The probe may be complementarily paired with the entire sequence of the first strand or part of the sequence of the first strand, and in one preferred embodiment, the entire sequence of the first strand is complementarily paired with the probe. It could be understood that the complementary pairing of the first strand with the probe is to ensure that the first strand preferentially hybridizes with the probe at the selected annealing temperature, and mismatches are allowed to be present in the paired sequence of the first strand with the probe when the above conditions are met.

For the purpose of effectively capturing a nucleic acid sequence comprising a first strand, the hybridization temperature T is set as Tm2 - 5 °C < T ≤ Tm1 - 5 °C, 80 °C ≥ (Tm1 - Tm2) ≥ 10 °C and 90 °C ≥ Tm1 ≥ 50 °C, where Tm1 is a melting temperature of the first strand, and Tm2 is a melting temperature of the second strand. In this way, an appropriate hybridization temperature could be obtained by optimization, the probability of hybridization of the second strand with the first strand can be reduced, thereby improving the hybridization efficiency of the probe with the first strand. Besides, the number of nucleic acid fragments with the same sequence (excluding the nucleic acid sequence of the adapter) is far less than the number of the probes, and the probability of forming double strands by pairing between single strands of the nucleic acid fragments at an annealing temperature is very low; therefore, the effect of the double strands formed between the nucleic acid fragments on the capture efficiency is negligible. In one embodiment of the present application, the Tm1 is 71 °C, the Tm2 is 45.6 °C, and the annealing temperature T is set as 55 °C.

The oligonucleotide strand in the probe is selected based on such factors as the annealing temperature, nucleotide composition, length and internal secondary structure of the oligonucleotide strand. In one embodiment of the present application, the oligonucleotide strand has a length of 20-80 nt.

Further, the nucleotide at the 3'-end of the oligonucleotide strand comprises a hydroxyl group (-OH), so that the 3'-end of the oligonucleotide strand can be linked to other nucleotides by a phosphodiester bond.

In one embodiment, the probe is immobilized on a solid medium selected from at least one of glass, plastic and magnetic beads. The immobilization of the probe on a solid medium, in one aspect, facilitates the separation and purification of a nucleic acid sequence captured by the probe, and in another aspect, enables other detections or operations, such as PCR amplification and sequencing, on the nucleic acid sequence captured by the probe.

In a ninth aspect, the present application provides a sequencing method comprising sequencing a hybrid complex obtained by the above method. In sequencing, when the hybrid complex is a complex comprising three sequences, a strand displacement DNA polymerase, such as Phi29 DNA polymerase, is adopted for sequencing; when the hybrid complex is a complex comprising two sequences, a DNA polymerase, such as Taq DNA polymerase, is commonly used for sequencing.

In a tenth aspect, the present application provides a kit for preparing a nucleic acid library, capture and/or sequencing, the kit comprising a first nucleic acid fragment, which is an adapter in one example. The kit further comprises a probe capable of capturing the first nucleic acid fragment, wherein the nucleotide at the 3'-end of the probe comprises -OH. It can be known from the complementary base pairing rule that, when the probe is paired with the first nucleic acid strand and the single-stranded end is located at the 3'-end of the first nucleic acid strand, the probe can be used as a primer, and the first nucleic acid strand can be used as a template for PCR amplification.

It will be understood that the oligonucleotide strand at one end of the probe is used for complementary hybridization with a sequence in a nucleic acid molecule, and the other end of the probe may be a nucleotide sequence or other sequences, such as an amino acid sequence, with other modifications, such as amino modifications and fluorescent modifications. In one example, the oligonucleotide strand is a DNA strand.

The oligonucleotide strand in the probe is selected based on such factors as the annealing temperature, nucleotide composition, length and internal secondary structure of the oligonucleotide strand. In one embodiment of the present application, the oligonucleotide strand has a length of 20-80 nt.

In one embodiment, the probe is immobilized on a solid medium selected from at least one of glass, plastic and magnetic beads.

### BRIEF DESCRIPTION OF THE DRAWINGS

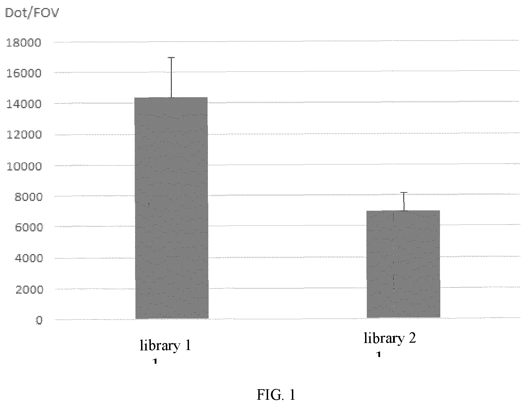

FIG. 1 illustrates the hybridization of library 1 and library 2 with probes in one embodiment of the present application, in which the abscissa represents library 1 and library 2, and the ordinate represents the number of dots within a specific field of view (FOV, e.g., 110 × 110 µm), which is expressed in Dot/FOV.

### DETAILED DESCRIPTION

The present application will be described below with reference to the specific examples and drawings. It is to be noted that these examples are intended to be illustrative only and are not to be construed as limiting the present application. The examples without a specified particular technique or condition are performed in accordance with techniques or conditions described in literatures in the art or in accordance with the product specification. The reagents or instruments not provided with manufacturer are conventional and commercially available products.

The terms "nucleic acid", "nucleic acid molecule" and "nucleic acid fragment" used herein include DNA, RNA, RNA-DNA and/or cDNA, which are double-stranded or single-stranded and are sequences consisting of nucleotides and/or nucleotide analogues (derivatives).

The terms "adapter" used herein is a nucleic acid fragment of known sequence.

### EMBODIMENTS

### 1. Adapter preparation

Nucleotide sequences shown in Table 1 were designed and synthesized.

**Table 1**

| name | Sequence (5'-3') | 5' modification | 3' modification |
|---|---|---|---|
| oligo 1.1 | AGATGTGTATAAGAGACAGT (SEQ ID NO.1) | | |
| oligo 1.2 | CTGTCTCTTATACACATCTGAGTGGAACTGGATGGTCGCAG GTATCAAGGA (SEQ ID NO.2) | 5'-PHO | 3'Cy3 |
| oligo 2.1 | TCCTTGATACCTGCGACCATCCAGTTCCACTCAGATGTGTA TAAGAGACAGT (SEQ ID NO.3) | | |
| oligo 2.2 | CTGTCTCTTATACACATCTGAGTGGAACTGGATGGTCGCAG GTATCAAGGA (SEQ ID NO.2) | 5'-PHO | 3'Cy3 |
| Probe | TTTTTTTTTTCCTTGATACCTGCGACCATCCAGTTCCACTC AGATGTGTATAAGAGACAG (SEQ ID NO.4) | 5'-NH₂C₆ | |

| | | | |
|---|---|---|---|
| Note: 5'-PHO: the 5'-end is modified by phosphorylation; 3' Cy3: the 3'-end is modified by Cy3; 5'-NH₂C₆: the 5'-end is modified by NH₂ at C6 of the nucleotide. | | | |

The melting temperature Tm for each strand was calculated using an online software (http://biotools.nubic.northwestern.edu/OligoCalc.html); the melting temperature of SEQ ID NO: 1 is 45.6 °C, and the melting temperature of SEQ ID NO: 2 is 71 °C.

The adapter preparation process: the synthesized nucleotide sequences shown in Table 1 were separately dissolved in 1 × Annealing Buffer (10 mM Tris, pH 7.5-8.0, 50 mM NaCl, 1 mM EDTA) to a concentration of 100 µmol/µL, and then quantified using Nanodrop 2000. According to the quantification results, the nucleotide sequences set forth in SEQ ID NOs: 1-4 were separately diluted to 100 µmol/µL with 1× Annealing Buffer. 10 µL of 100 µmol/µL oligo1.1 and oligo1.2 were pipetted into a clean 200 µL PCR tube, and well mixed with pipette to prepare adapter 1. Similarly, 10 µL of 100 µmol/µL oligo2.1 and oligo2.2 were pipetted into another PCR tube, and well mixed to prepare adapter 2. The mixed sequences were slowly annealed in a PCR machine to prepare adapter 1 and adapter 2 under the conditions shown in Table 2:

**Table 2**

| Temperature | Time | Cycle |
|---|---|---|
| 95 °C | 3 min | |
| 95 °C | 3s | 790 cycles, a decrease of 0.1 °C in temperature after each cycle, with the rate of heat transfer between cycles set at 0.1 °C/s. |
| 12 °C | Hold | |

The prepared adapters 1 and 2 were quantified using a Qubit 2.0 quantifier and Qubit^{™} dsDNA BR Assay Kit.

### 2. Library construction

DNA fragment repair and adapter ligation were performed using a kit from Vazyme (VAHTS Universal DNA library prep kit for Illumina, Cat. # ND606), wherein the molar ratio of DNA fragments to adapters was 1:200, i.e., DNA:adapter = 1:200. Library 1 was constructed using adapter 1 and library 2 was constructed using adapter 2. The library 1 and library 2 were constructed under the same conditions except that the adapters used were different. The specific steps were as follows:

### Step (1): end repair

Fragmented DNAs with a length of 100-500 bp (main band at 200 bp) were filled-in at ends, phosphorylated at the 5'-ends and dA-tailed at the 3'-ends.

An end fill-in reaction system was prepared in a sterile PCR tube, and the specific composition is shown in Table 3:

**Table 3**

| Component | Volume |
|---|---|
| DNA (38 nM) | 10 µL |
| End Prep Mix 4 | 15 µL |
| ddH₂O | To 65 µL |

The end fill-in reaction system was well mixed and then briefly centrifuged to cause the reaction system to settle to the bottom of the tube.

The end-repair conditions are shown in Table 4:

**Table 4**

| Temperature | Time |
|---|---|
| Heated lid at 105 °C | On |
| 20 °C | 15 min |
| 65 °C | 15 min |
| 4 °C | Hold |

After the reaction was completed, an end-repair product was obtained.

### Step (2): adapter ligation

The end-repair product in the step (1) was ligated to the adapter.
(1) An adapter ligation reaction system was prepared in the PCR tube for end repair in step (1) and shown in Table 5:

**Table 5**

| Component | Volume |
|---|---|
| End-repair product | 65 µL |
| Rapid Ligation buffer 2 | 25 µL |
| Rapid DNA ligase | 5 µL |
| Adapter 1 or 2 (15 µM) | 5 µL |
| Total | 100 µL |

The reaction system was gently pipetted to be well mixed and briefly centrifuged to cause the reaction system to settle to the bottom of the PCR tube.

(2) The PCR tube for the adapter ligation reaction system prepared in step (1) was placed in a PCR machine for adapter ligation reaction under the conditions shown in Table 6:

**Table 6**

| Temperature | Time |
|---|---|
| Heated lid at 105 °C | On |
| 20 °C | 15 min |
| 4 °C | Hold |

After the reaction was completed, an adapter ligation product was obtained.

### Step (3): purification

The adapter ligation product in step (2) was purified using VAHTS DNA Clean Beads according to the following steps:
a) after the magnetic beads were equilibrated to room temperature, VAHTS DNA Clean Beads were vortexed to be well mixed;
b) 60 µL of VAHTS DNA Clean Beads were pipetted into 100 µL of the adapter ligation product, and the resulting mixture was vortexed or pipetted 10 times to be well mixed;
c) incubation at room temperature for 5 min;
d) the PCR tube was briefly centrifuged and placed on a magnetic rack to separate the magnetic beads from the liquid, and after the solution cleared (about 5 min), the supernatant was carefully removed;
e) with the PCR tube kept on the magnetic rack all the time, 200 µL of freshly prepared 80% ethanol was added to rinse the magnetic beads, and after 30 s of incubation at room temperature, the supernatant was carefully removed;
f) step e) was repeated for a total of two rinses;
g) with the PCR tube kept on the magnetic rack all the time, the lid was opened, and the magnetic beads were dried in the air for 5-10 min until no ethanol remained; and
h) the PCR tube was removed from the magnetic rack and eluted:
   22.5 µL of eluent (10 mM Tris-HCl, pH 8.0-8.5) was added for elution, and the resulting mixture was vortexed or gently pipetted to be well mixed and left to stand at room temperature for 2 min; the PCR tube was briefly centrifuged and left to stand on a magnetic rack, and after the solution cleared (about 5 min), 20 µL of the supernatant was transferred to a new EP tube without touching the magnetic beads.

The purified libraries were quantified using a Qubit 2.0 quantifier and QubitTM dsDNA BR Assay Kit, wherein the adapter used for library 1 was adapter 1, and the adapter used for library 2 was adapter 2.

### 3. Hybrid capture

A probe (SEQ ID NO: 4) was immobilized on a chip by the method disclosed in the specification of published Patent Application No. CN201510501968.7, and the prepared libraries 1 and 2 were diluted with 3× SSC hybridization solution and then hybridized with the probe immobilized on the chip. The number of the adapter sequences hybridized with the probe was then determined according to Cy3 signals.

The procedure for library-chip hybridization was as follows:
(1) Chip selection: the substrate glass of the chip used was an epoxy-modified glass chip from SCHOTT; a probe (SEQ ID NO: 4) was immobilized by reacting amino groups on the probe with epoxy groups on the chip surface, e.g., by the method disclosed in published Patent Application No. CN201811191589.2, with the immobilized probe density being 18,000 Dot/FOV in a region of 110× 110 µm, i.e., there were 18,000 dots in a 1 10×110 µm field of view.
(2) Hybridization solution preparation: as shown in Table 7, the hybridization solution was prepared from 20× SSC buffer (Sigma, # S6639-1L) having a final concentration of 3× SSC and a library having a concentration of 1 nM, with a total volume of 40 µL. The prepared hybridization solution was denatured at 95 °C for 2 min, and then quickly transferred onto ice for cooling.

**Table 7**

| | |
|---|---|
| 20× SSC buffer | 6 µL |
| Library 1 or library 2 | Final concentration of 1 nM |
| Nucleic acid-free water | Making up to 40 µL |

(3) The denatured hybridization solution was quickly loaded onto the chip, which was then left to stand at 55 °C for 30 min to allow hybridization of the library with the probe on the chip surface.
(4) The chip was washed with 3× SSC, 1× SSC and 0.1× SSC in sequence.
(5) The chip was then placed on a sequencer and photographed at 532 mm wavelength, and the number of dots in each field of view was counted.

### 4. Experimental results

The experimental results are shown in FIG. 1, in which the abscissa represents library 1 and library 2, and the ordinate represents Dot/FOV (the number of dots observed in a region of 110 × 110 µm). The comparison between the hybridization results of library 1 and library 2 shows that, under the condition of the same hybridization concentration and field of view (a region of 110 × 110 µm), the hybridization density of library 1 (14,400 dots on average) was much greater than that of library 2 (7000 dots on average), indicating that the hybridization efficiency of library 1 with the probe was higher than that of library 2 with the probe.

Reference throughout this specification to "an embodiment", "one embodiment", "another embodiment", "some embodiments", "an example", "a specific example", "another example", or "some examples", means that a particular feature, structure, material, or characteristic described in connection with the embodiment or example is included in at least one embodiment or example of the present disclosure. Thus, the appearances of the phrases such as "in an embodiment", "in one embodiment", "in another embodiment", "in some embodiments", "in an example", "in a specific example", "in another example", or "in some examples", in various places throughout this specification are not necessarily referring to the same embodiment or example of the present disclosure. Furthermore, the particular features, structures, materials, or characteristics may be combined in any suitable manner in one or more embodiments or examples. In addition, in the absence of contradiction, those skilled in the art can combine the different embodiments or examples described in this specification, or combine the features of different embodiments or examples.

## Claims

1. A sequencing method comprising:
preparing a nucleic acid library, comprising ligating a first nucleic acid fragment and a second nucleic acid fragment to obtain the nucleic acid library, wherein
the first nucleic acid fragment and the second nucleic acid fragment are both double-stranded DNAs, the first nucleic acid fragment is a known sequence and comprises a first strand and a second strand that are complementary; the first nucleic acid fragment has a single-stranded end located at the 3'-end of the first strand, and 80 °C ≥ (Tm1 - Tm2) ≥ 10 °C and 90 °C ≥ Tm1 ≥ 50 °C, wherein Tm1 is a melting temperature of the first strand, and Tm2 is a melting temperature of the second strand;
capturing the nucleic acid library, comprising denaturing the nucleic acid library to obtain a single-stranded product and hybridizing the single-stranded product with a probe to obtain a hybrid complex or capturing the undenatured nucleic acid library with a probe to obtain a hybrid complex, wherein Tm2 - 5 °C ≤ T ≤ Tm1 - 5 °C, wherein T is a temperature for the hybridization;
sequencing the hybrid complex.

2. The method according to claim 1, wherein the Tm1 is 71 °C, and the Tm2 is 45.6 °C.

3. The method according to claim 1 or 2, wherein the 5'-end of the first strand has a phosphate group; and/or the 3'-end of the first strand has an optically detectable label, wherein optionally the optically detectable label is a fluorescent molecule; and/or the 3'-end of the second strand has no hydroxyl group.

4. The method according to any of claims 1 to 3, wherein the ligation is selected from one of blunt-end ligation and sticky-end ligation; and/or
mixing the first nucleic acid fragment and the second nucleic acid fragment at a molar ratio of 50:1 to 500:1 to perform the ligation, wherein optionally the molar ratio is 200:1.

5. A method for capturing a nucleic acid molecule, comprising:
1) denaturing a nucleic acid library to obtain a single-stranded product, wherein the nucleic acid library is prepared according to the method of any of claims 1 to 4; and
2) hybridizing the single-stranded product in step 1) with a probe to obtain a hybrid complex.

6. The method according to claim 5, wherein the probe comprises a single-stranded oligonucleotide, wherein a nucleotide at the 3'-end of the single-stranded oligonucleotide comprises a hydroxyl group; and/or
the probe is complementary to the entire sequence of the first strand.

7. The method according to claim 5 or 6, Tm2 - 5 °C ≤ T ≤ Tm1 - 5 °C, wherein T is a temperature for the hybridization; optionally wherein the single-stranded oligonucleotide has a length of 20-80 nt; optionally wherein the probe is immobilized on a solid base selected from at least one of glass, plastic and magnetic beads.

8. A kit for preparing a nucleic acid library, capture and/or sequencing according to the method of claim 1 to 7, the kit comprising a first nucleic acid fragment, wherein the first nucleic acid fragment is a double-stranded DNA of known sequence, comprises a first strand and a second strand that are complementary, and has a single-stranded end; and 80 °C ≥ (Tm1 - Tm2) ≥ 10 °C and 90 °C ≥ Tm1 ≥ 50 °C,
wherein Tm1 is a melting temperature of the first strand, and Tm2 is a melting temperature of the second strand, wherein
the first strand is 5'-CTGTCTCTTATACACATCTGAGTGGAACTGGATGGTCGCAGGTATCAAGGA-3' (SEQ ID NO.2)
the second strand is 5'-AGATGTGTATAAGAGACAGT-3' (SEQ ID NO.1).

9. The kit according to claim 8, wherein the single-stranded end is located at the 3'-end of the first strand; optionally
the Tm1 is 71 °C, and the Tm2 is 45.6 °C.

10. The kit according to claim 8, wherein the 5'-end of the first strand has a phosphate group; optionally the 3'-end of the first strand has an optically detectable label, wherein optionally the optically detectable label is a fluorescent molecule.

11. The kit according to claim 8, wherein the 3'-end of the second strand has no hydroxyl group.

12. The kit according to any of claims 8 to 11, further comprising a probe capable of capturing the first nucleic acid fragment, wherein the probe comprises a single-stranded oligonucleotide, and a nucleotide at the 3'-end of the single-stranded oligonucleotide comprises -OH; optionally wherein the single-stranded oligonucleotide has a length of 20-80 nt.

13. The kit according to claim 12, wherein the probe is immobilized on a solid base selected from at least one of glass, plastic and magnetic beads.

14. A use of the kit as claimed in claims any of 8 to 13 in sequence hybridization and/or sequencing.

## Patentansprüche

1. Sequenzierungsverfahren, umfassend:
Herstellen einer Nukleinsäurebibliothek, umfassend das Ligieren eines ersten Nukleinsäurefragments und eines zweiten Nukleinsäurefragments, um die Nukleinsäurebibliothek zu erhalten, wobei
das erste Nukleinsäurefragment und das zweite Nukleinsäurefragment beide doppelsträngige DNAs sind, wobei das erste Nukleinsäurefragment eine bekannte Sequenz ist und einen ersten Strang und einen zweiten Strang umfasst, die komplementär sind; das erste Nukleinsäurefragment ein am 3'-Ende des ersten Strangs befindliches einzelsträngiges Ende aufweist, und 80 °C ≥ (Tm1 - Tm2) ≥ 10 °C und 90 °C ≥ Tm1 ≥ 50 °C, wobei Tm1 eine Schmelztemperatur des ersten Strangs ist und Tm2 eine Schmelztemperatur des zweiten Strangs ist;
Einfangen der Nukleinsäurebibliothek, umfassend das Denaturieren der Nukleinsäurebibliothek, um ein einzelsträngiges Produkt zu erhalten, und Hybridisieren des einzelsträngigen Produkts mit einer Sonde, um einen Hybridkomplex zu erhalten, oder Einfangen der nicht denaturierten Nukleinsäurebibliothek mit einer Sonde, um einen Hybridkomplex zu erhalten, wobei Tm2 -5 °C ≤ T ≤ Tm1 - 5 °C, wobei T eine Temperatur für die Hybridisierung ist;
Sequenzieren des Hybridkomplexes.

2. Verfahren nach Anspruch 1, wobei die Tm1 71 °C und die Tm2 45,6 °C beträgt.

3. Verfahren nach Anspruch 1 oder 2, wobei das 5'-Ende des ersten Strangs eine Phosphatgruppe aufweist; und/oder das 3'-Ende des ersten Strangs eine optisch nachweisbare Markierung aufweist, wobei die optisch nachweisbare Markierung gegebenenfalls ein fluoreszierendes Molekül ist; und/oder das 3'-Ende des zweiten Strangs keine Hydroxylgruppe aufweist.

4. Verfahren nach einem der Ansprüche 1 bis 3, wobei die Ligation aus einer Blunt-End-Ligation und einer Sticky-End-Ligation ausgewählt ist; und/oder Mischen des ersten Nukleinsäurefragments und des zweiten Nukleinsäurefragments in einem molaren Verhältnis von 50:1 bis 500:1, um die Ligation durchzuführen, wobei das molare Verhältnis gegebenenfalls 200:1 beträgt.

5. Verfahren zum Einfangen eines Nukleinsäuremoleküls, umfassend:
1) Denaturieren einer Nukleinsäurebibliothek, um ein einzelsträngiges Produkt zu erhalten, wobei die Nukleinsäurebibliothek nach dem Verfahren nach einem der Ansprüche 1 bis 4 hergestellt wird; und
2) Hybridisieren des einzelsträngigen Produkts in Schritt 1) mit einer Sonde, um einen Hybridkomplex zu erhalten.

6. Verfahren nach Anspruch 5, wobei die Sonde ein einzelsträngiges Oligonukleotid umfasst, wobei ein Nukleotid am 3'-Ende des einzelsträngigen Oligonukleotids eine Hydroxylgruppe umfasst; und/oder die Sonde komplementär zur gesamten Sequenz des ersten Strangs ist.

7. Verfahren nach Anspruch 5 oder 6, Tm2 - 5 °C ≤ T ≤ Tm1 - 5 °C, wobei T eine Temperatur für die Hybridisierung ist, gegebenenfalls wobei das einzelsträngige Oligonukleotid eine Länge von 20-80 nt aufweist, gegebenenfalls wobei die Sonde auf einem festen Träger immobilisiert ist, der aus mindestens einem von Glas-, Kunststoff- und magnetischen Perlen ausgewählt ist.

8. Kit zum Herstellen einer Nukleinsäurebibliothek, Einfangen und/oder Sequenzieren nach dem Verfahren nach Anspruch 1 bis 7, wobei das Kit ein erstes Nukleinsäurefragment umfasst, wobei das erste Nukleinsäurefragment eine doppelsträngige DNA bekannter Sequenz ist, einen ersten Strang und einen zweiten Strang umfasst, die komplementär sind, und ein einzelsträngiges Ende aufweist; und 80 °C ≥ (Tm1 - Tm2) ≥ 10 °C und 90 °C > Tm1 ≥ 50 °C, wobei Tm1 eine Schmelztemperatur des ersten Strangs ist und Tm2 eine Schmelztemperatur des zweiten Strangs ist, wobei
der erste Strang 5'-CTGTCTCTTATACACATCTGAGTGGAACTGGATGGTCGCAGGTATCAAGGA -3' (SEQ ID NO.2) ist
der zweite Strang 5'-AGATGTGTATAAGAGACAGT-3' (SEQ ID NO.1) ist.

9. Kit nach Anspruch 8, wobei sich das einzelsträngige Ende am 3'-Ende des ersten Strangs befindet; gegebenenfalls wobei
die Tm1 71 °C beträgt und die Tm2 45,6 °C beträgt.

10. Kit nach Anspruch 8, wobei das 5'-Ende des ersten Strangs eine Phosphatgruppe aufweist; gegebenenfalls wobei das 3'-Ende des ersten Strangs eine optisch nachweisbare Markierung aufweist, wobei die optisch nachweisbare Markierung gegebenenfalls ein fluoreszierendes Molekül ist.

11. Kit nach Anspruch 8, wobei das 3'-Ende des zweiten Strangs keine Hydroxylgruppe aufweist.

12. Kit nach einem der Ansprüche 8 bis 11, ferner umfassend eine Sonde, die das erste Nukleinsäurefragment einfangen kann, wobei die Sonde ein einzelsträngiges Oligonukleotid umfasst, und ein Nukleotid am 3'-Ende des einzelsträngigen Oligonukleotids -OH umfasst; wobei das einzelsträngige Oligonukleotid gegebenenfalls eine Länge von 20-80 nt aufweist.

13. Kit nach Anspruch 12, wobei die Sonde auf einem festen Träger immobilisiert ist, der aus mindestens einem von Glas-, Kunststoff- und magnetischen Perlen ausgewählt ist.

14. Verwendung des Kits nach einem der Ansprüche 8 bis 13 zur Sequenzhybridisierung und/oder Sequenzierung.

## Revendications

1. Procédé de séquençage comprenant :
la préparation d'une banque d'acides nucléiques, comprenant la ligature d'un premier fragment d'acide nucléique et d'un deuxième fragment d'acide nucléique pour obtenir la banque d'acides nucléiques,
le premier fragment d'acide nucléique et le deuxième fragment d'acide nucléique étant tous deux des ADN double brin, le premier fragment d'acide nucléique étant une séquence connue et comprenant un premier brin et un deuxième brin qui sont complémentaires ; le premier fragment d'acide nucléique ayant une extrémité simple brin située à l'extrémité 3' du premier brin, et 80 °C ≥ (Tm1 - Tm2) ≥ 10 °C et 90 °C ≥ Tm1 ≥ 50 °C, Tm1 étant une température de fusion du premier brin, et Tm2 étant une température de fusion du deuxième brin ;
la capture de la banque d'acides nucléiques, comprenant la dénaturation de la banque d'acides nucléiques pour obtenir un produit simple brin et l'hybridation du produit simple brin avec une sonde pour obtenir un complexe hybride ou la capture de la banque d'acides nucléiques non dénaturée avec une sonde pour obtenir un complexe hybride, où Tm2 - 5 °C ≤ T ≤ Tm1 - 5 °C, T étant une température pour l'hybridation ;
le séquençage du complexe hybride.

2. Procédé selon la revendication 1, dans lequel Tm1 est de 71 °C, et Tm2 est de 45,6 °C.

3. Procédé selon la revendication 1 ou 2, dans lequel l'extrémité 5' du premier brin a un groupe phosphate ; et/ou l'extrémité 3' du premier brin a un marqueur optiquement détectable, le marqueur optiquement détectable étant éventuellement une molécule fluorescente ; et/ou l'extrémité 3' du deuxième brin n'ayant pas de groupe hydroxyle.

4. Procédé selon l'une quelconque des revendications 1 à 3, dans lequel la ligature est choisie parmi la ligature à extrémité franche et la ligature à extrémité collante ; et/ou le mélange du premier fragment d'acide nucléique et du deuxième fragment d'acide nucléique en un rapport molaire de 50:1 à 500:1 pour effectuer la ligature, le rapport molaire étant éventuellement de 200:1.

5. Procédé de capture d'une molécule d'acide nucléique, comprenant :
1) la dénaturation d'une banque d'acides nucléiques pour obtenir un produit simple brin, la banque d'acides nucléiques étant préparée conformément au procédé selon l'une quelconque des revendications 1 à 4 ; et
2) l'hybridation du produit simple brin de l'étape 1) avec une sonde pour obtenir un complexe hybride.

6. Procédé selon la revendication 5, dans lequel la sonde comprend un oligonucléotide simple brin, un nucléotide à l'extrémité 3' de l'oligonucléotide simple brin comprenant un groupe hydroxyle ; et/ou la sonde est complémentaire de la séquence entière du premier brin.

7. Procédé selon la revendication 5 ou 6, Tm2 - 5 °C ≤ T ≤ Tm1 - 5 °C, où T est une température pour l'hybridation ; éventuellement dans lequel l'oligonucléotide simple brin a une longueur de 20 à 80 nt ; éventuellement dans lequel la sonde est immobilisée sur une base solide choisie parmi au moins une parmi le verre, le plastique et les billes magnétiques.

8. Kit pour la préparation d'une banque d'acides nucléiques, la capture et/ou le séquençage conformément au procédé selon les revendications 1 à 7, le kit comprenant un premier fragment d'acide nucléique, le premier fragment d'acide nucléique étant un ADN double brin de séquence connue, comprenant un premier brin et un deuxième brin qui sont complémentaires, et ayant une extrémité simple brin ; et 80 °C ≥ (Tm1 - Tm2) ≥ 10 °C et 90 °C ≥ Tm1 ≥ 50 °C, où Tm1 est une température de fusion du premier brin, et Tm2 est une température de fusion du deuxième brin, où
le premier brin est 5'-CTGTCTCTTATACACATCTGAGTGGAACTGGATGGTCGCAGGTATCAAGGA -3' (SEQ ID NO. 2)
le deuxième brin est 5'-AGATGTGTATAAGAGACAGT-3' (SEQ ID NO. 1).

9. Kit selon la revendication 8, dans lequel l'extrémité simple brin est située à l'extrémité 3' du premier brin ; éventuellement.
Tm1 est de 71 °C et Tm2 de 45,6 °C.

10. Kit selon la revendication 8, dans lequel l'extrémité 5' du premier brin a un groupe phosphate ; éventuellement.
l'extrémité 3' du premier brin a un marqueur optiquement détectable, le marqueur optiquement détectable étant éventuellement une molécule fluorescente.

11. Kit selon la revendication 8, dans lequel l'extrémité 3' du deuxième brin n'a pas de groupe hydroxyle.

12. Kit selon l'une quelconque des revendications 8 à 11, comprenant en outre une sonde capable de capturer le premier fragment d'acide nucléique, dans lequel la sonde comprend un oligonucléotide simple brin, et un nucléotide à l'extrémité 3' de l'oligonucléotide simple brin comprend -OH ; éventuellement dans lequel l'oligonucléotide simple brin a une longueur de 20 à 80 nt.

13. Kit selon la revendication 12, dans lequel la sonde est immobilisée sur une base solide choisie parmi au moins une parmi le verre, le plastique et les billes magnétiques.

14. Utilisation du kit selon l'une quelconque des revendications 8 à 13 pour l'hybridation et/ou le séquençage.
